# EUROPEAN PATENT SPECIFICATION

(11) **EP 3 610 935 B1**
(45) Date of publication and mention of the grant of the patent: **30.12.2020**
(21) Application number: 19196809.8
(22) Date of filing: 05.11.2018
(51) Int. Cl.: B01D 11/02, B01D 11/04, B01D 35/18, B01D 24/00, B01D 29/56

(54) **SYSTEM FOR STAGED CONTINUOUS COOLED FILTRATION**
SYSTEM ZUR GESTUFTEN KONTINUIERLICHEN GEKÜHLTEN FILTRATION
SYSTÈME DE FILTRATION REFROIDIE CONTINUE ÉTAGÉE

(30) Priority: 10.11.2017 US 201715809980
(43) Date of publication of application: 19.02.2020
(62) Document number(s) of the earlier application(s) in accordance with Art. 76 EPC: 18204438.8 / 3 482 808
(73) Proprietor: NextLeaf Solutions Ltd., Vancouver, British Columbia V6B 1A4 (CA)
(72) Inventor: KO, Ryan Delmoral, Coquitlam, British Columbia V3K 7B1 (CA)
(74) Representative: Kalkoff & Partner Patentanwälte PartmbB

(56) References cited:
- WO-A1-2017/015065
- JP-A- S60 211 287
- US-A1- 2016 303 490
- US-A1- 2017 014 731
- US-A1- 2017 043 276
- US-B2- 9 655 937

## Description

### TECHNICAL FIELD

This application relates to filtering. More specifically, it relates to a multi-stage, chilled filter arranged in a closed-loop configuration.

### BACKGROUND

Filtering is a necessary step in the process of essential element extraction. Traditionally, the vertically oriented in-line and T-form filter housings that exist require the main body of the housing to be lifted off the base in order to replace the filter cartridge inside.

In legal, adult-use markets, sales of cannabis extracts are growing ten times faster compared to the sales of dried cannabis, and extracts account for over 60% of revenue. With legalization, consumer preferences are shifting from dried cannabis to extracted cannabis products. However, the scent and flavors of cannabis can be undesirable in many infused products because of excess lipids, plant matter and impurities present in currently available extracts.

U.S. Patent No. 9,155,767 to Hospodor et al. relates to the extraction of medicinal cannabis compounds into an eluate, by separating a portion of medicinal cannabis compounds contained within a portion of eluate at a first extraction target level, to provide enough clean solvent to continue extraction operations. A high efficiency concentrator processes eluate from one or more tanks, creating clean solvent when extraction targets are met or when clean solvent is exhausted. This manages eluate concentration levels and limits the quantity of concentrated medicinal cannabis compounds on site at any moment in time.

U.S. Patent No. 9,655,937 to Jones discloses extraction devices, methods, and systems. Example devices have a solvent chamber, a plant material chamber, a collection chamber, and a solvent return that create a sealed, closed-cycle extraction and/or solvent purification process. Any extractable plant material can be used in the disclosed devices, methods, and systems although in some examples some form of the cannabis plant is used.

US patent application No. 2017014731 of Chess discloses an extraction system which can be used to recover light oils and other organic materials during a supercritical or subcritical fluid extraction. The extraction system may include a dual purpose pressure vessel which can be used as an independent, full spectrum extractor by adding organic material within a compartment within the vessel and passing liquefied, compressed gases onto and through organic material as well as a separator to separate the extracting fluid from the extracted material.

This background information is provided to reveal information believed by the applicant to be of possible relevance to the present invention.

### SUMMARY OF INVENTION

The present invention is directed to a system of chilled filters connected in a closed-loop configuration for use in the extraction of essential elements from plant material. When extracting essential elements, the plant material is washed and/or soaked in a solvent such as ethanol to result in a crude oil and ethanol mixture, for example. The system of chilled filters is used to filter the crude oil and solvent mixture to remove particulate matter, and may be used at different stages of the extraction process. In particular, the filter system may be used as part of a process for extracting cannabinoids from cannabis plants, for example for medicinal purposes.

Disclosed herein is a filtration system comprising a solvent vessel, a plurality of filter stages and the same plurality of transfer tubes. Each filter stage has a thermally insulated cylindrical side wall; a lid that is removable from said wall; a replaceable, elongated filter cartridge; a support configured to locate a base of said filter cartridge centrally in the filter stage; an inlet port in the lid located to introduce liquid into the filter cartridge; an inlet port configured for introducing pressurized gas outside of the filter cartridge; and a base with an outlet port that drains fluid from outside the filter cartridge. The solvent vessel has a thermally insulated side wall; an inlet port configured for introducing pressurized gas; and an outlet tube having a first end located at a bottom region of the solvent vessel and a second end located outside the solvent vessel. The transfer tubes are each removably connected at an outlet end thereof to one of the inlet ports in one of the lids of the filter stages; and at an inlet end thereof to either the second end of the outlet tube of the solvent vessel or the outlet port of another of said filter stages such that the solvent vessel and the filter stages are connected in series.

### BRIEF DESCRIPTION OF DRAWINGS

The following drawings illustrate embodiments of the invention, which should not be construed as restricting the scope of the invention in any way.
FIG. 1 is a schematic diagram of the closed-loop, multi-stage, chilled filter system, according to an embodiment of the present invention.
FIG. 2 is a flowchart of a process undertaken using the system of FIG. 1.
FIG. 3 is a schematic diagram of an alternate embodiment of the closed-loop, multi-stage, chilled filter system of the present invention.

### DESCRIPTION

### A. Glossary

Cannabinoids are a group of chemicals that act on cannabinoid receptors in the body, numerous of which are found in the cannabis plant. Cannabidiol (CBD) is one of the active cannabinoids found in cannabis and is used for medicinal purposes. Tetrahydrocannabinol (THC) is a psychotropic cannabinoid and is the main psychoactive ingredient of cannabis. THC also has medicinal uses. THCa is the non-psychoactive form of THC.

Crude oil is a term for the description of condensed, non-filtered oil, i.e. oil that is non-winterized and not treated via charcoal, clay and silica. The crude oil contains the essential elements. Winterization refers to the removal of unwanted plant waxes and lipids.

### B. Apparatus

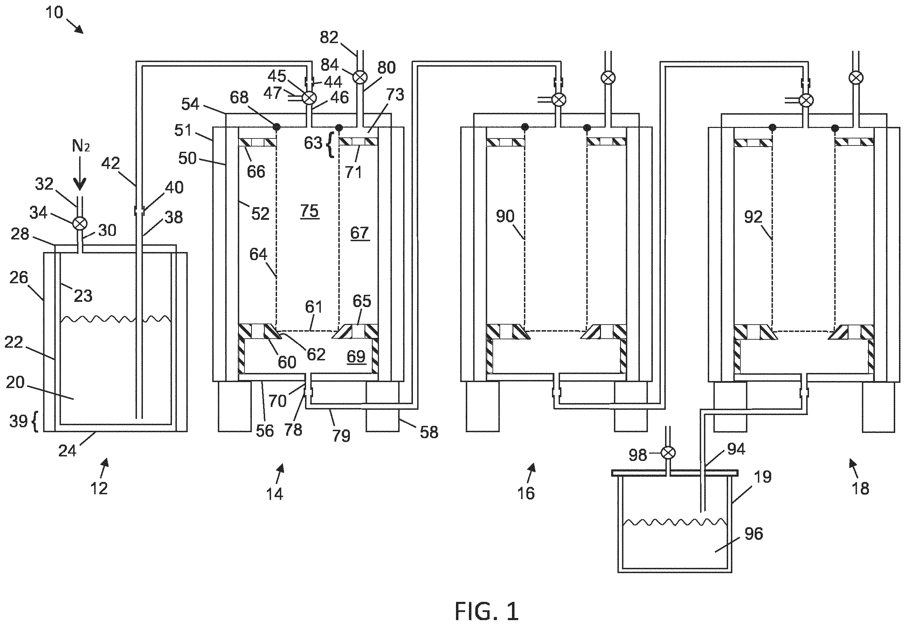

Referring to FIG. 1, a closed-loop, multi-stage filter system 10 is shown, which includes a solvent vessel 12, a first filter stage 14, a second filter stage 16 and a third filter stage 18 all connected in series, and a collection vessel 19 for collecting filtered liquids from the system.

The solvent vessel 12 contains the crude oil and solvent mixture 20 that is to be filtered. The crude oil and solvent mixture 20 is the liquid that is drawn out of an extractor column, for example, i.e. after the plant material in the extractor column has been washed and/or soaked with the solvent. The crude oil contains essential elements and is dissolved in the solvent. The mixture also contains some unwanted residual plant matter and other undesirable components. Before the crude oil and solvent mixture 20 is placed in the solvent vessel 12, it may undergo one or more pre-filtering steps. For example, the crude oil and solvent mixture 20 may be pre-filtered with activated charcoal, clay and/or silica. The charcoal removes pigments, chlorophyll, heavy metals and particulates. The clay primarily removes pigments. The silica removes very fine plant matter and other particulates.

The stainless steel solvent vessel 12 has an outer wall 22, an inner wall 23 and a base 24. The outer wall 22 and inner wall 23 form a side wall or jacket that is filled with chilled, pressurized liquid CO₂ in order to keep the contents of the solvent vessel 12 cool. Thermal insulation 26 is wrapped around the outer wall 22 of the filter stage 12. In other embodiments, the jacket could be evacuated to provide thermal insulation, and the thermal insulation is not necessary. The main requirement is that the inner wall 23 of the solvent vessel be thermally insulated from ambient temperatures of the surrounding atmosphere in order to keep the contents of the vessel cool. Optionally, the jacket includes the base 24 of the solvent vessel 12, and the base may be further thermally insulated.

A removable lid 28 seals to the top of the solvent vessel 12 so that the contents of the vessel can be pressurized via inlet port 30. Nitrogen gas under pressure is fed into the vessel 12 via inlet tube 32 and valve 34. As the solvent vessel 12 is pressurized, the crude oil and solvent mixture 20 within it is forced out of the vessel through exit tube 38, which forms an airtight seal around its outside with the lid 28. The exit tube has an inlet in the bottom region 39 of the solvent vessel 12 so that liquid in the solvent vessel can readily enter it.

The exit tube 38 is connected via an airtight connector 40 outside of the solvent vessel 12 to a transfer tube 42. The transfer tube 42 is connected in turn via an airtight connector 44 and 3-way valve 45 to the inlet port 46 of the first filtering stage 14. Optionally, the transfer tube 42 is thermally insulated. Additional solvent may be introduced via the inlet tube 47 connected to the 3-way valve 45.

The first filter stage 14 has an outer side wall 50 surrounded by a thermal insulator 51, an inner side wall 52 spaced apart from the outer side wall, a lid 54 that seals to the top of the filter stage and a base 56. The outer wall 50 and inner wall 52 form a side wall or jacket that is filled with chilled, pressurized liquid CO₂ in order to keep the contents of the filter stage 14 cool. While different configurations of thermal insulation are possible, the main requirement is that the inner wall 52 of the filter stage 14 is thermally insulated from ambient temperatures in order to keep the contents of the stage cool. Optionally, the jacket includes the base 56 of the filter stage 14, and the base may be further thermally insulated. The first stage 14 is sealed against the atmosphere so that it can be pressurized. The first stage 14 is mounted on supports 58.

A support 60, which is positioned in the bottom of the filter stage 14, has a locating feature such as a beveled edge 62. The locating feature 62 serves to position the lower portion or base 61 of a replaceable cylindrical filter cartridge 64 centrally in the filter stage 14. In other embodiments, different shapes of the support are possible. In this embodiment, the filter cartridge is a polypropylene filter with a pore size in the range of 10-15µm. Other filter sizes may be used in other embodiments. At the top of the filter stage 14 there is a guide ring 66, which serves to direct the filter cartridge 64 along the axis of the filter stage and maintain an upper portion 63 of the filter cartridge aligned axially within the filter stage. In other embodiments the shape of the guide ring 66 is different.

The support 60 has through holes 65 to permit the passage of filtered liquid from a volume 67 of the filter stage above the support to a volume 69 below it, which is adjacent to and in fluid communication with the outlet port 70. The guide 66 has through holes 71 to permit the passage of filtered liquid from a volume 73 above the guide to the volume 67 below the guide. Supports 60 and guides 66 may have slots or gaps with other shapes to provide fluid communication between the volumes 73, 67, 69 of the solvent vessel. The top of the filter cartridge 64 is sealed to the underside of the lid 54 with an O-ring 68.

The crude oil and solvent mixture 20 enters the filter stage 14 through inlet port 46, which directs the mixture into the inner region 75 of the filter cartridge 64. The mixture is then filtered as it passes out through the side walls and base of the filter cartridge 64. The filtered mixture collects in the bottom of the filter stage 14 and passes out of it through exit port 70 in the base 56 of the filter stage.

When required, the cartridge 64 is replaced by disconnecting transfer tube 42 from the lid 54, removing the lid from the filter stage 14, lifting out the cartridge, and then placing a new cartridge in its place. By removing only the lid 54 from the filter stage 14, the side walls 50, 52 can remain in place, together with the thermal insulation 51. This makes it convenient to change the filter cartridge 64, particularly if the filter stage is large. In some embodiments, the side walls 50 can be 1m tall or more. As well as making the filter cartridge more convenient to change, taller filtration stages can be used within the same headroom compared to stages that require the outer walls to be lifted to change the filter cartridge.

A connector 78 connects a second transfer tube 79 to the outlet port 70 of the filter stage 14. A second port 80 in the lid 54 of the filter stage 14 allows for nitrogen to be supplied directly to the filter stage through tube 82 and valve 84. This is useful in case a blockage occurs upstream in the system 10.

The transfer tube 74 is connected via an airtight connector to the inlet port of the second filtering stage 16. The second filter stage 16 is similar to the first filtering stage 14, except that the filter cartridge 90 has a smaller pore size, which in this embodiment is in the range 3-10µm. Other filter sizes may be used in other embodiments.

The outlet port at the bottom of the second filter stage 16 is connected via a third transfer tube to an inlet port in the top of the lid of the third filtering stage 18. The third filter stage 18 is similar to the first and second filter stages 14, 16, except that the filter cartridge 92 has an even smaller pore size, which in this embodiment is 1µm. Other filter sizes may be used in the third filter stage 18 in other embodiments, including filter sizes that are smaller than 1µm.

The outlet port at the bottom of the third filter stage 18 is connected via a connector and collection pipe 94 to the collection vessel 19, in which the filtered crude oil and solvent mixture 96 is collected. The collection vessel 19 is covered or sealed from the atmosphere with a pressure relief valve 98, although, optionally, it may be uncovered.

A benefit of having the solvent vessel 12 and the three filter stages 14, 16, 18 sealed from the atmosphere is that it reduces the amount of condensation of water into the chilled solvent. This would otherwise dilute the solvent and reduce its effectiveness.

### C. Exemplary Process

In use, the crude oil and solvent mixture is chilled either before placing it in the solvent vessel 12 or while it is in the solvent vessel. After this, nitrogen is fed into the solvent vessel 12 at a pressure in the range of about 70-210kPa (10-30psi). When the filter system 10 is used for the extraction of essential elements from cannabis and the solvent used is ethanol, the crude oil and solvent mixture is maintained at a temperature between -40°C and -20°C in the solvent vessel 12, and remains below -10°C as it passes through the three filter stages 14, 16, 18.

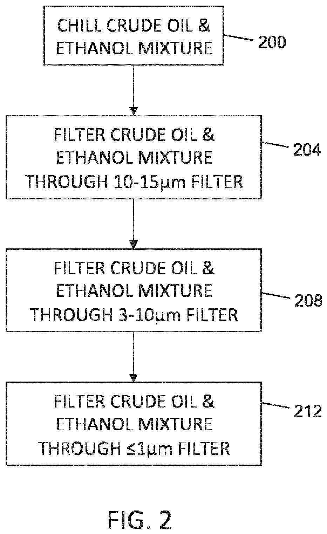

Referring to FIG. 2, the first step of the process is to chill the crude oil and ethanol mixture in step 200 to a temperature between -40°C and -20°C. In step 204, the crude oil and ethanol mixture is filtered through a 10-15µm filter in the first filtering stage 14. In step 208, the crude oil and ethanol mixture is then filtered through a 3-10µm filter in the second filtering stage 16. In step 212, the crude oil and ethanol mixture is finally filtered through a ≤1µm filter in the third filtering stage 18.

### D. Variations

While the best presently contemplated mode of carrying out the subject matter disclosed and claimed herein has been described, other modes are also possible.

The outlet tube 38 of the solvent vessel 12 may lead downwards from the base 24 of the solvent vessel, in the same way that the outlet ports 70 are located on the filter stages 14, 16, 18.

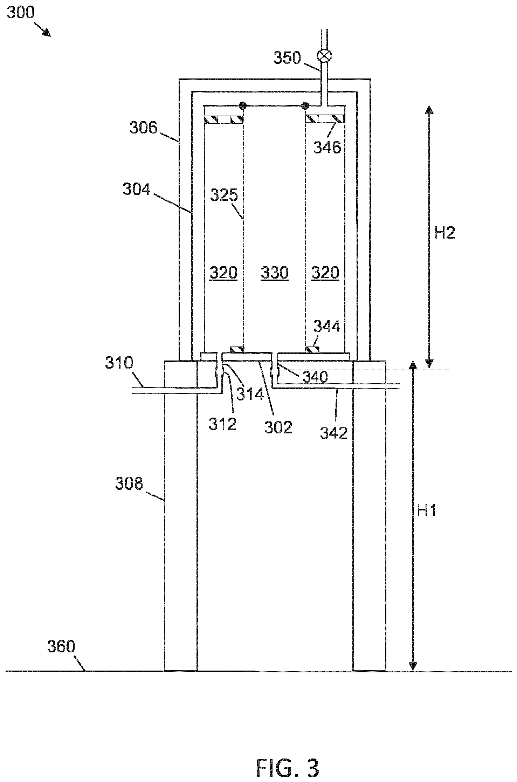

Referring to FIG. 3, an alternate filter stage 300 is shown. Multiple such filter stages can be connected in series as above. The filter stage 300 includes a removable base 302 with jacketed sidewall 304 and surrounding insulation 306, mounted on legs 308 or other equivalent rack or support. An inlet pipe 310 from the previous stage or from the solvent vessel feeds fluid to be filtered into the stage, via the connector 312 and inlet port 314. The fluid to be filtered is fed into the volume 320 to the outside of the filter cartridge 325. As the fluid is filtered, it passes through the cartridge 325 to the volume 330 on the inside of the cartridge, and then leaves the filter stage via outlet port 340 in the removable base 302. Outlet pipe 342 transfers the filtered fluid to the next stage or to the collection vessel 19. The cartridge 325 is aligned with a guide 344 on the removable base 302 and with a spacer 346 at the upper end of the cartridge. A further inlet port 350 is used to introduce nitrogen gas if needed and/or solvent. Other valve and/or inlets may be included in the filter stage 300, e.g. for evacuating the jacket, filling it with chilled liquid CO₂, removing fluids from the stage and/or for introducing fluids into the stage.

The legs 308 or other equivalent support holds the body or main sidewall 304 of the filter stage 300 off the floor by a distance that is sufficient to detach the inlet and outlet pipes 310, 342 from the removable base 302, lower the base and remove/replace the cartridge 325 without having to move the sidewall 304 H1. This is possible when distance H1 is greater than distance H2. Distance H1 is the height of the legs off the floor 360 and distance H2 is the height of the cartridge 325 plus the height of the removable base 302 with its permanent fixtures (i.e. inlet port 314 and outlet port 340). It is important not to have to disturb the jacketed sidewall 304 so that the insulation 306 does not need to be moved.

In some embodiments, the apparatus is portable so that it can be taken to the different sites of various plant growers, to be used on an as-needed basis.

Different filter media may be used in this apparatus, such as clay, charcoal and silica.

In other embodiments within the purview of the present invention, other plant materials besides cannabis may be processed. For example, lavender and hemp may be processed, as well as other plants that produce phytochemicals of interest, which include cannabinoids, terpenes, and flavonoids.

Temperatures that have been given to the nearest degree include all temperatures within a range of ±0.5°C of the given value.

In general, unless otherwise indicated, singular elements may be in the plural and vice versa with no loss of generality.

Throughout the description, specific details have been set forth in order to provide a more thorough understanding of the invention. However, the invention may be practiced without these particulars. In other instances, well known elements have not been shown or described in detail and repetitions of steps and features have been omitted to avoid unnecessarily obscuring the invention. For example, various ports, valves, tubes and other thermal insulation are not shown for clarity. Accordingly, the specification and drawings are to be regarded in an illustrative, rather than a restrictive, sense.

It will be clear to one having skill in the art that further variations to the specific details disclosed herein can be made, resulting in other embodiments that are within the scope of the invention disclosed. All parameters, dimensions, proportions, relative proportions, materials, and configurations described herein are examples only and may be changed depending on the specific embodiment. Accordingly, the scope of the invention is defined by the following claims.

## Claims

1. A filtration system comprising a solvent vessel (12), a plurality of filter stages (14, 16, 18) and the same plurality of transfer tubes (42, 74, 79), wherein:
the solvent vessel (12) has:
a thermally insulated side wall (22, 23);
an inlet port (30) for introducing pressurized gas; and
an outlet tube (38) having a first end located at a bottom region of the solvent
vessel (12) and a second end located outside the solvent vessel (12);
and the transfer tubes (42, 74, 79) are each removably connected:
at an outlet end thereof to one of the inlet ports (46) in one of the lids of the filter stages (14, 16, 18); and
at an inlet end thereof to either the second end of the outlet tube (38) of the solvent vessel (12) or the outlet port (70) of another of said filter stages such that the solvent vessel (12) and the filter stages (14, 16, 18) are connected in series, **characterized in that** each filter stage (14, 16, 18) has:
a thermally insulated cylindrical side wall (50, 52);
a lid (28, 54) that is removable from said wall;
a replaceable, elongated filter cartridge (64, 90, 92);
a support having a cartridge locating surface that positions a base of said filter cartridge (64, 90, 92) centrally in the filter stage (14, 16, 18);
an inlet port (46) in the lid (54) located to introduce liquid into the filter cartridge (64, 90, 92);
an inlet port (80) located to introduce pressurized gas into the filter stage (12, 14, 16, 18) outside of the filter cartridge (64, 90, 92); and
a base with an outlet port (70) that drains fluid from outside the filter cartridge (64, 90, 92).

2. The filtration system of claim 1 further comprising a guide (66) positioned to locate an upper portion (63) of the filter cartridge (64, 90, 92) centrally in the filter stage (14, 16, 18).

3. The filtration system of claim 1 further comprising a seal between each filter cartridge (64, 90, 92) and the lid (54) of the corresponding filter stage (14, 16, 18).

4. The filtration system of claim 1, wherein each support (60) defines a fluid communication path from a volume (67) above the support (60) to a volume (69) adjacent the outlet port (70).

5. The filtration system of claim 2 wherein each guide (66) defines a fluid communication path (71) from a volume (73) above the guide (66) to a volume (67) below the guide (66).

6. The filtration system of claim 1, wherein there are three filter stages (14, 16, 18) and:
a first of the filter stages (14) is connected to the solvent vessel (12) and its filter cartridge (64) has a pore size of 10-15µm;
a second of the filter stages (16) is connected to the first filter stage (14) and its filter cartridge (90) has a pore size of 3-10µm;
the third of the filter stages (18) is connected to the second filter stage (16) and its filter cartridge (92) has a pore size of ≤1µm; and
each filter cartridge is made from polypropylene.

7. The filtration system of claim 1, wherein the solvent vessel (12) has chilled liquid carbon dioxide in its side wall (22, 23).

8. The filtration system of claim 1, wherein each filter stage (14, 16, 18) has chilled liquid carbon dioxide in its side wall (50, 52).

9. The filtration system of claim 1, wherein the pressurized gas is nitrogen that is pressurized to 70-210kPa.

10. The filtration system of claim 1, wherein the filtration system is sealed from an external atmosphere everywhere except at the outlet port of the filter stage (18) that is at an end of the series.

11. The filtration system of claim 10, wherein the outlet port of the filter stage (18) that is at the end of the series discharges via a collection pipe into a collection vessel (19).

12. The filtration system of claim 1, wherein:
the outlet port of the filter stage (18) that is at the end of the series discharges via a collection tube (19) into a collection vessel (19); and
the collection vessel (19) is sealed from an external atmosphere via a pressure relief valve (98).

13. The filtration system of claim 1, wherein:
the transfer tubes are thermally insulated; and
the bases of the filter stages (18) and a base of the solvent vessel (12) are thermally insulated from an external atmosphere.

14. The filtration system of claim 1, wherein:
the side walls (50, 52) of one or both of the filter stages (18) and the solvent vessel (12) are evacuated; and
the bases of the filter stages (18) and a base of the solvent vessel (12) are double walled and are either evacuated or filled with liquid carbon dioxide.

15. The filtration system of claim 1, wherein each filter stage (14, 16, 18) comprises a further inlet located to introduce solvent into the filter cartridge (64, 90, 92).

## Patentansprüche

1. Filtrationssystem, das einen Lösungsmittelbehälter (12), eine Vielzahl von Filterstufen (14, 16, 18) und die gleiche Vielzahl von Transferleitungen (42, 74, 79) umfasst, wobei:
der Lösungsmittelbehälter (12) Folgendes aufweist:
eine thermisch isolierte Seitenwand (22, 23);
eine Einlassöffnung (30) zum Einlassen von Druckgas; und
eine Auslassleitung (38), die ein erstes Ende, das sich an einem Bodenbereich des Lösungsmittelbehälters (12) befindet, und ein zweites Ende aufweist, das sich außerhalb des Lösungsmittelbehälters (12) befindet;
und die Transferleitungen (42, 74, 79) jeweils lösbar verbunden sind:
an einem Auslassende davon mit einer von den Einlassöffnungen (46) in einem von den Deckeln der Filterstufen (14, 16, 18); und
an einem Einlassende davon mit entweder dem zweiten Ende der Auslassleitung (38) des Lösungsmittelbehälters (12) oder der Auslassöffnung (70) einer anderen der Filterstufen, derart, dass der Lösungsmittelbehälter (12) und die Filterstufen (14, 16, 18) in Reihe verbunden sind,
**dadurch gekennzeichnet, dass** jede Filterstufe (14, 16, 18) Folgendes aufweist:
eine thermisch isolierte zylindrische Seitenwand (50, 52);
einen Deckel (28, 54), der von der Wand abnehmbar ist;
einen austauschbaren länglichen Filtereinsatz (64, 90, 92);
ein Stützelement, das eine Oberfläche zur Positionierung des Einsatzes aufweist,
die eine Basis des Filtereinsatzes (64, 90, 92) zentral in der Filterstufe (14, 16, 18) positioniert;
eine Einlassöffnung (46) in dem Deckel (54), die so gelegen ist, dass sie Flüssigkeit in den Filtereinsatz (64, 90, 92) einführt;
eine Einlassöffnung (80), die so gelegen ist, dass sie Druckgas in die Filterstufe (12, 14, 16, 18) außerhalb des Filtereinsatzes (64, 90, 92) einführt; und
eine Basis mit einer Auslassöffnung (70), die Fluid von außerhalb des Filtereinsatzes (64, 90, 92) ablässt.

2. Filtrationssystem nach Anspruch 1, das ferner eine Führung (66) umfasst, die positioniert ist, um einen oberen Abschnitt (63) des Filtereinsatzes (64, 90, 92) zentral in der Filterstufe (14, 16, 18) anzuordnen.

3. Filtrationssystem nach Anspruch 1, das ferner eine Dichtung zwischen jedem Filtereinsatz (64, 90, 92) und dem Deckel (54) der entsprechenden Filterstufe (14, 16, 18) umfasst.

4. Filtrationssystem nach Anspruch 1, wobei jedes Stützelement (60) einen Fluidkommunikationspfad von einem Volumen (67) über dem Stützelement (60) zu einem Volumen (69) angrenzend an die Auslassöffnung (70) bestimmt.

5. Filtrationssystem nach Anspruch 2, wobei jede Führung (66) einen Fluidkommunikationspfad (71) von einem Volumen (73) über der Führung (66) zu einem Volumen (67) unter der Führung (66) bestimmt.

6. Filtrationssystem nach Anspruch 1, wobei drei Filterstufen (14, 16, 18) vorhanden sind und:
eine erste der Filterstufen (14) mit dem Lösungsmittelbehälter (12) verbunden ist und ihr Filtereinsatz (64) eine Porengröße von 10 bis 15 µm aufweist;
eine zweite der Filterstufen (16) mit der ersten Filterstufe (14) verbunden ist und ihr Filtereinsatz (90) eine Porengröße von 3 bis 10 µm aufweist;
die dritte der Filterstufen (18) mit der zweiten Filterstufe (16) verbunden ist und ihr Filtereinsatz (92) eine Porengröße von ≤ 1 µm aufweist; und
jede Filterstufe aus Polypropylen hergestellt ist.

7. Filtrationssystem nach Anspruch 1, wobei der Lösungsmittelbehälter (12) gekühltes flüssiges Kohlendioxid in seiner Seitenwand (22, 23) aufweist.

8. Filtrationssystem nach Anspruch 1, wobei jede Filterstufe (14, 16, 18) gekühltes flüssiges Kohlendioxid in ihrer Seitenwand (50, 52) aufweist.

9. Filtrationssystem nach Anspruch 1, wobei das Druckgas Stickstoff ist, das auf 70 bis 210 kPa druckbeaufschlagt ist.

10. Filtrationssystem nach Anspruch 1, wobei das Filtrationssystem gegenüber einer äußeren Atmosphäre überall abgedichtet ist, außer an der Auslassöffnung der Filterstufe (18), die sich an einem Ende der Reihe befindet.

11. Filtrationssystem nach Anspruch 10, wobei die Auslassöffnung der Filterstufe (18), die sich an dem Ende der Reihe befindet, über eine Sammelrohrleitung in einen Sammelbehälter (19) ablässt.

12. Filtrationssystem nach Anspruch 1, wobei:
die Auslassöffnung der Filterstufe (18), die sich an dem Ende der Reihe befindet, über eine Sammelleitung (19) in einen Sammelbehälter (19) ablässt; und
der Sammelbehälter (19) über ein Überdruckventil (98) gegenüber einer äußeren Atmosphäre abgedichtet ist.

13. Filtrationssystem nach Anspruch 1, wobei:
die Transferleitungen thermisch isoliert sind; und
die Basen der Filterstufen (18) und eine Basis des Lösungsmittelbehälters (12) gegenüber einer äußeren Atmosphäre thermisch isoliert sind.

14. Filtrationssystem nach Anspruch 1, wobei:
die Seitenwände (50, 52) von einer oder beiden von den Filterstufen (18) und dem Lösungsmittelbehälter (12) evakuiert sind; und
die Basen der Filterstufen (18) und eine Basis des Lösungsmittelbehälters (12) doppelwandig sind und entweder evakuiert oder mit flüssigem Kohlendioxid gefüllt sind.

15. Filtrationssystem nach Anspruch 1, wobei jede Filterstufe (14, 16, 18) einen weiteren Einlass umfasst, der so gelegen ist, dass er Lösungsmittel in den Filtereinsatz (64, 90, 92) einführt.

## Revendications

1. Système de filtration comprenant un récipient à solvant (12), une pluralité d'étages de filtre (14, 16, 18) et la même pluralité de tubes de transfert (42, 74, 79), dans lequel :
- le récipient à solvant (12) comporte :
- une paroi latérale isolée thermiquement (22, 23) ;
- un orifice d'entrée (30) pour l'introduction de gaz sous pression ; et
- un tube de sortie (38) comportant une première extrémité située au niveau d'une région de fond du récipient à solvant (12) et une deuxième extrémité située à l'extérieur du récipient à solvant (12) ;
- et les tubes de transfert (42, 74, 79) sont reliés respectivement de façon détachable :
- par une extrémité de sortie de ceux-ci à l'un des orifices d'entrée (46) dans l'un des couvercles des étages de filtre (14, 16, 18) ; et
- par une extrémité d'entrée de ceux-ci, soit à la deuxième extrémité du tube de sortie (38) du récipient à solvant (12) ou à l'orifice de sortie (70) d'un autre parmi lesdits étages de filtre, de telle façon que le récipient à solvant (12) et les étages de filtre (14, 16, 18) sont reliés en série,
**caractérisé en ce que** chaque étage de filtre (14, 16, 18) comporte :
- une paroi latérale cylindrique isolée thermiquement (50, 52) ;
- un couvercle (28, 54) détachable de ladite paroi ;
- une cartouche de filtre allongée remplaçable (64, 90, 92) ;
- un support comportant une surface de positionnement de cartouche, permettant de positionner une base de ladite cartouche de filtre (64, 90, 92) de façon centrale dans l'étage de filtre (14, 16, 18) ;
- un orifice d'entrée (46) dans le couvercle (54), positionné pour introduire du liquide dans la cartouche de filtre (64, 90, 92) ;
- un orifice d'entrée (80) positionné pour introduire du gaz sous pression dans l'étage de filtre (12, 14, 16, 18) à l'extérieur de la cartouche de filtre (64, 90, 92) ; et
- une base avec un orifice de sortie (70) drainant du fluide depuis l'extérieur de la cartouche de filtre (64, 90, 92) .

2. Système de filtration selon la revendication 1, comprenant en outre un guide (66) agencé pour positionner une partie supérieure (63) de la cartouche de filtre (64, 90, 92) de façon centrale dans l'étage de filtre (14, 16, 18).

3. Système de filtration selon la revendication 1, comprenant en outre un joint d'étanchéité entre chaque cartouche de filtre (64, 90, 92) et le couvercle (54) de l'étage de filtre (14, 16, 18) correspondant.

4. Système de filtration selon la revendication 1, dans lequel chaque support (60) définit un chemin de communication fluidique à partir d'un volume (67) situé au-dessus du support (60) vers un volume (69) adjacent à l'orifice de sortie (70).

5. Système de filtration selon la revendication 2, dans lequel chaque guide (66) définit un chemin de communication fluidique (71) à partir d'un volume (73) situé au-dessus du guide (66) vers un volume (67) situé en dessous du guide (66) .

6. Système de filtration selon la revendication 1, dans lequel il est prévu trois étages de filtre (14, 16, 18) et :
- un premier parmi les étages de filtre (14) est relié au récipient à solvant (12) et sa cartouche de filtre (64) présente une taille de pores entre 10 et 15 µm ;
- un deuxième parmi les étages de filtre (16) est relié au premier étage de filtre (14) et sa cartouche de filtre (90) présente une taille de pores entre 3 et 10 µm ;
- le troisième parmi les étages de filtre (18) est relié au deuxième étage de filtre (16) et sa cartouche de filtre (92) présente une taille de pores ≤ 1 µm ; et
- chaque cartouche de filtre est constituée de polypropylène.

7. Système de filtration selon la revendication 1, dans lequel le récipient à solvant (12) comporte du dioxyde de carbone liquide refroidi dans sa paroi latérale (22, 23).

8. Système de filtration selon la revendication 1, dans lequel chaque étage de filtre (14, 16, 18) comporte du dioxyde de carbone liquide refroidi dans sa paroi latérale (50, 52).

9. Système de filtration selon la revendication 1, dans lequel le gaz sous pression est du nitrogène qui est mis sous pression entre 70 et 210 kPa.

10. Système de filtration selon la revendication 1, dans lequel le système de filtration est étanche à une atmosphère externe, partout sauf au niveau de l'orifice de sortie de l'étage de filtre (18) situé à une extrémité de la série.

11. Système de filtration selon la revendication 10, dans lequel l'orifice de sortie de l'étage de filtre (18) situé à l'extrémité de la série se décharge par le biais d'un tuyau de collecte vers un récipient de collecte (19).

12. Système de filtration selon la revendication 1, dans lequel :
- l'orifice de sortie de l'étage de filtre (18) situé à l'extrémité de la série se décharge par le biais d'un tube de collecte (19) vers un récipient de collecte (19) ; et
- le récipient de collecte (19) est étanche à une atmosphère externe par le biais d'une soupape de décharge de pression (98).

13. Système de filtration selon la revendication 1, dans lequel :
- les tubes de transfert sont isolés thermiquement ; et
- les bases des étages de filtre (18) et une base du récipient à solvant (12) sont isolées thermiquement par rapport à une atmosphère externe.

14. Système de filtration selon la revendication 1, dans lequel :
- les parois latérales (50, 52) de l'un ou des deux parmi les étages de filtre (18) et le récipient à solvant (12) sont évacués ; et
- les bases des étages de filtre (18) et une base du récipient à solvant (12) présentent des doubles parois et sont évacuées ou remplies avec du dioxyde de carbone liquide.

15. Système de filtration selon la revendication 1, dans lequel chaque étage de filtre (14, 16, 18) comprend une entrée supplémentaire positionnée pour introduire du solvant dans la cartouche de filtre (64, 90, 92).
